# EUROPEAN PATENT APPLICATION

(11) **EP 4 548 882 A1**
(43) Date of publication of application: **07.05.2025**
(21) Application number: 24209129.6
(22) Date of filing: 28.10.2024
(51) Int. Cl.: A61F 2/46

(54) **IMPLANT EXTRACTOR**

(30) Priority: 03.11.2023 US 202363595812 P
(71) Applicant: Shukla Medical, St. Petersburg, FL 33709 (US)
(72) Inventor: GOSIK-WOLFE, Adam, Auburndale, FL 33823 (US); KISKA, Mitchell, Seminole, FL 33772 (US)
(74) Representative: Swea IP Law AB

(57) **Abstract**

An implant extractor (100) for extracting an implant from bone comprising a housing (120), an adjustment mechanism (130) within the housing, an outer (150) sleeve extending from the housing, an inner shaft (160) within the outer sleeve and having a proximal end operatively engaged with the adjustment mechanism, and a clamp (170) operatively engaged with distal ends of the inner shaft and the outer sleeve.

## Description

### FIELD OF THE INVENTION

The subject disclosure relates generally to the field of orthopedic implant extraction devices, systems, and methods.

### BACKGROUND OF THE DISCLOSURE

Surgical extraction of a joint implant from bone may be medically indicated for reasons including, for example, infection, implant fracture, or recurrent dislocation. The present invention provides an improved device and method for removing an elbow implant or other bone implants, which reduces or avoids torque on the implant during extraction and, thereby, possible injury to the patient's bone or soft tissue.

### SUMMARY OF THE DISCLOSURE

Exemplary embodiments of the subject disclosure relate generally to a surgical extraction tool and, more specifically, to a tool for extracting an implant from bone, such as an elbow implant, among others. More particularly, an implant extractor of the present disclosure is a tool which is for extracting the humeral and/or ulnar component of an elbow implant or other implants from other bones. The present disclosure further provides methods for extracting a bone implant from a patient.

In an exemplary embodiment of the subject disclosure, the implant extractor comprises a housing, an adjustment mechanism within the housing, an outer sleeve extending from the housing, an inner shaft within the outer sleeve and having a proximal end operatively engaged with the adjustment mechanism, and a clamp operatively engaged with distal ends of the inner shaft and the outer sleeve.

In an aspect of the subject disclosure, the inner shaft includes a threaded proximal end. According to another aspect, the inner shaft includes a slot and the outer sleeve includes a shaft for moving within the slot.

In another aspect, the adjustment mechanism comprises a threaded stem for engaging the threaded proximal end of the inner shaft. According to another aspect, the outer sleeve includes a shoulder about its proximal end abutting the housing. According to another aspect, the outer sleeve includes a shoulder about its distal end abutting the clamp.

In another aspect, the clamp comprises a base and a foot. According to another aspect, the base has a concave face. According to another aspect, the concave face is a substantially V-shaped face. According to another aspect, the concave face is substantially V-shaped in two directions. According to another aspect, the concave face includes a textured surface. According to another aspect, the textured surface includes ribs, teeth, knurling, protrusions, grooves, ridges, dimples, spikes, or a combination thereof. According to another aspect, the foot is tapered. According to another aspect, the foot includes a textured surface.

According to an aspect, the housing has a window in communication with the proximal portion of the adjustment mechanism. According to another aspect, the implant extractor further comprises a quick connect attached to a proximal end of the housing.

According to an aspect, one of the outer sleeve and the inner shaft moves relative to the other of the outer sleeve and the inner shaft upon operation of the adjustment mechanism. According to another aspect, the outer sleeve moves relative to the inner shaft upon operation of the adjustment mechanism.

Other features and advantages of the subject disclosure will be apparent from the following more detailed description of the exemplary embodiments.

### BRIEF DESCRIPTION OF THE SEVERAL VIEWS OF THE DRAWINGS

The foregoing summary, as well as the following detailed description of the exemplary embodiments of the subject disclosure, will be better understood when read in conjunction with the appended drawings. For the purpose of illustrating the present disclosure, there are shown in the drawings exemplary embodiments. It should be understood, however, that the subject application is not limited to the precise arrangements and instrumentalities shown.
FIG. 1 is a side view of an implant extractor in accordance with an exemplary embodiment of the present disclosure;
FIG. 2 is a longitudinal cross-sectional view of the implant extractor of FIG. 1 shown engaging an elbow implant;
FIGS. 3A and 3B are exploded views of the exemplary implant extractor of FIG. 1;
FIGS. 4A-4C depict an elbow implant implanted in a patient's ulnar bone;
FIG. 5 is an elevation view of a quick connect of the implant extractor of FIG. 1;
FIG. 6 is a side view of a housing of the implant extractor of FIG. 1;
FIG. 7 is a side view of an adjustment mechanism of the implant extractor of FIG. 1;
FIG. 8 is a side view of an outer sleeve of the implant extractor of FIG. 1;
FIGS. 9A and 9B are perspective views of an inner shaft of the implant extractor of FIG. 1;
FIGS. 10 A-10C are various views of a clamp of the exemplary implant extractor of FIG. 1;
FIG 11 is an enlarged perspective view of a pan head screw of the implant extractor of FIG. 1;
FIG. 12 is an enlarged perspective view of a threaded pin of the implant extractor of FIG. 1;
FIG. 13 is an enlarged perspective view of a clamp foot of the implant extractor of FIG. 1; and
FIGS. 14A and 14B are a proximal perspective view and a distal perspective view, respectively, of a clamp base of the implant extractor of FIG. 1.

### DETAILED DESCRIPTION

Reference will now be made in detail to an exemplary embodiment of the subject disclosure illustrated in the accompanying drawings. Wherever possible, the same or like reference numbers will be used throughout the drawings to refer to the same or like features. It should be noted that the drawings are in simplified form and are not drawn to precise scale. In reference to the disclosure herein, for purposes of convenience and clarity only, directional terms such as upper, lower, top, bottom, above, below and diagonal, are used with respect to the accompanying drawings. Such directional terms used in conjunction with the following description of the drawings should not be construed to limit the scope of the subject disclosure in any manner not explicitly set forth. Additionally, the term "a," as used in the specification, means "at least one." The terminology includes the words above specifically mentioned, derivatives thereof, and words of similar import.

"About" as used herein when referring to a measurable value such as an amount, a temporal duration, and the like, is meant to encompass variations of ±20%, ±10%, ±5%, ±1%, or ±0.1% from the specified value, as such variations are appropriate.

"Substantially" as used herein shall mean considerable in extent, largely but not wholly that which is specified, or an appropriate variation therefrom as is acceptable within the field of art. "Exemplary" as used herein shall mean serving as an example.

Throughout the subject application, various aspects thereof can be presented in a range format. It should be understood that the description in range format is merely for convenience and brevity and should not be construed as an inflexible limitation on the scope of the subject disclosure. Accordingly, the description of a range should be considered to have specifically disclosed all the possible subranges as well as individual numerical values within that range. For example, description of a range such as from 1 to 6 should be considered to have specifically disclosed subranges such as from 1 to 3, from 1 to 4, from 1 to 5, from 2 to 4, from 2 to 6, from 3 to 6 etc., as well as individual numbers within that range, for example, 1, 2, 2.7, 3, 4, 5, 5.3, and 6. This applies regardless of the breadth of the range.

Furthermore, the described features, advantages and characteristics of the exemplary embodiments of the subject disclosure may be combined in any suitable manner in one or more embodiments. One skilled in the relevant art will recognize, in light of the description herein, that the subject disclosure can be practiced without one or more of the specific features or advantages of a particular exemplary embodiment. In other instances, additional features and advantages may be recognized in certain embodiments that may not be present in all exemplary embodiments of the present disclosure.

The present disclosure provides an implant extraction device or extractor that enables a user to clamp the extractor onto an elbow implant or other implant implanted in a patient's bone and pull the implant extraction device in order to remove the implant from the bone.

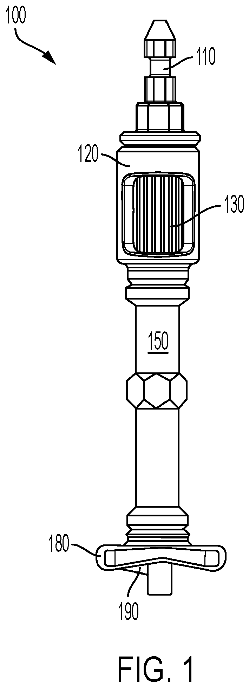

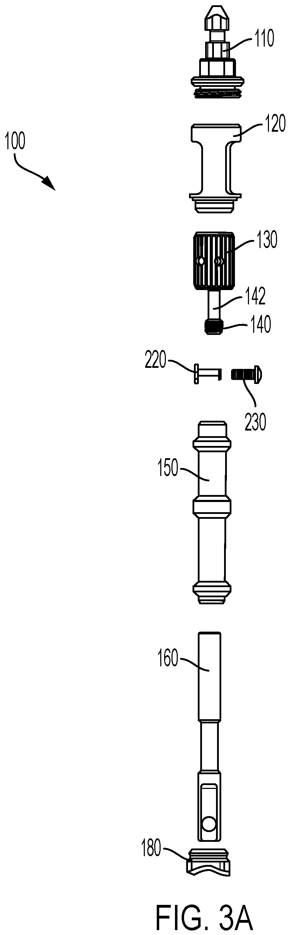

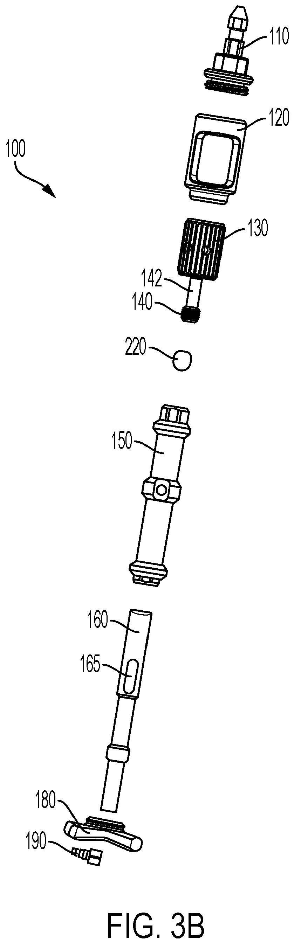

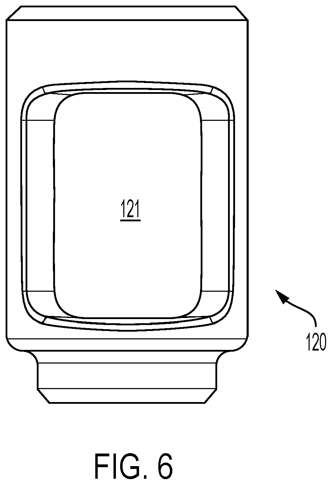

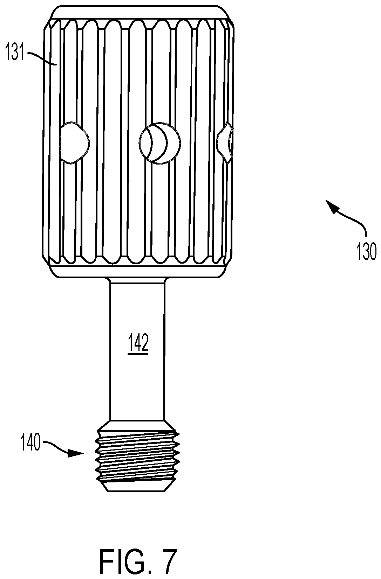

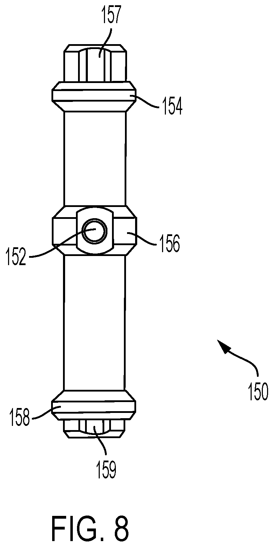

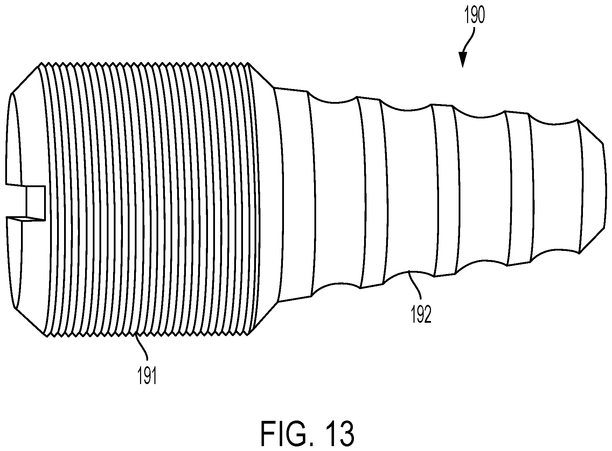

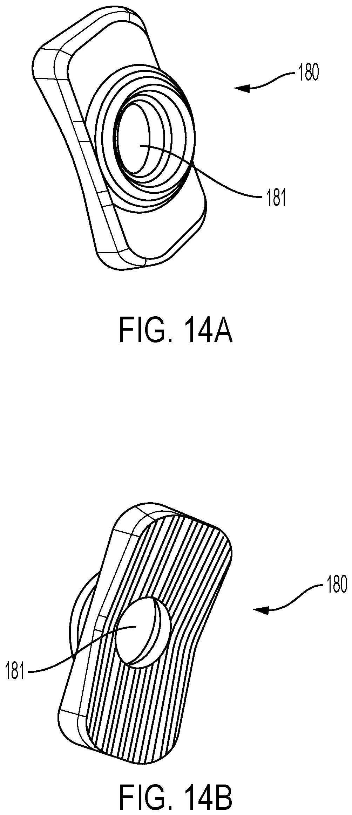

Referring to the drawings, FIGS. 1-3 and 5-14 show an exemplary embodiment of an implant extractor 100 in accordance with the subject disclosure. The implant extractor includes a housing 120, an adjustment mechanism 130, an outer sleeve 150, an inner shaft 160 and a clamp 170. A quick connect 110 is attached to and extends from a proximal end of the housing 120. The adjustment mechanism 130 is within the housing, and the outer sleeve 150 extends from the housing. The inner shaft 160 is within the outer sleeve and has a proximal end operatively engaged with the adjustment mechanism. The clamp 170 is operatively engaged with distal ends of the inner shaft and the outer sleeve.

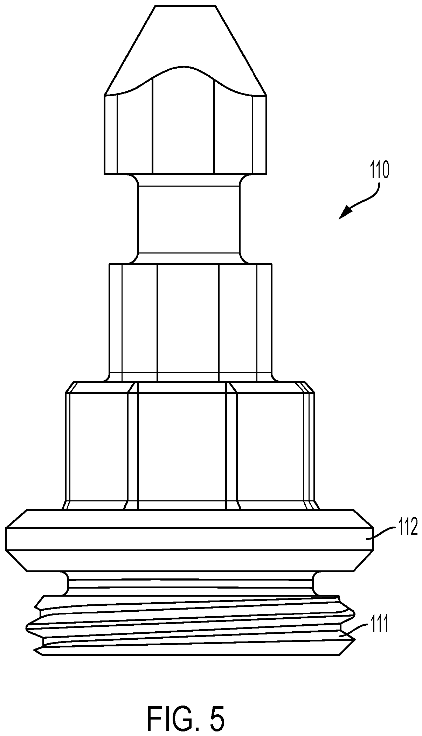

The quick connect 110 is configured as best shown in FIGS. 1, 2, 3A, 3B and 5. The quick connect 110 includes a threaded base 111 (FIG. 5) at its distal end for threadedly connecting to the housing 120. The base of the quick connect also includes a flange 112 for abutting a proximal end of the housing.

The housing 120 is configured as best shown in FIGS. 1, 2, 3A, 3B and 6. The housing 120 is a substantially annular or tubular housing having openings at its proximal and distal ends. The housing 120 further includes at least one window 121 in a lateral wall thereof in communication with a proximal portion of the adjustment mechanism mounted therein and which permits a user to access and rotate the adjustment mechanism 130. The housing includes threads about its proximal end opening to receive the threaded distal end or base 111 of the quick connection 110.

The adjustment mechanism 130 is configured as best shown in FIGS. 1, 2, 3A, 3B and 7. A proximal portion 131 of the adjustment mechanism 130 is substantially cylindrical with outer lateral surfaces being textured e.g., with grooves, ribs, teeth, knurling, protrusions, grooves, ridges, dimples, spikes, or a combination thereof to facilitate its rotation by a user. A distal portion of the adjustment mechanism 130 includes an externally threaded stem 140. The stem 140 has a smaller diameter than the proximal portion 131 and extends distally via a shaft 142 from a distal end of the proximal portion. The stem 140 is sized and configured to reside within the outer sleeve 150 and the inner shaft 160, as shown in FIGS. 1, 2, 3A and 3B. As described in greater detail below, the stem 140 threadedly engages the inner shaft 160. The stem 140 is formed such that its external threads have an overall diameter larger than an overall diameter of the shaft 142.

In operation, as the proximal portion 131 of the adjustment mechanism 130 is rotated by a user, the stem 140 rotates whereby its threads operatively engages the threads on the inner shaft 160. During use, the clamp engages an implant to be extracted, as such the clamp and inner shaft are fixed in position relative to the implant it is attached to. Thus, as the stem 140 rotates, the outer sleeve moves relative to the inner shaft upon operation of the adjustment mechanism. That is, both the adjustment mechanism and the outer sleeve move relative to the inner shaft. More particularly, the adjustment mechanism and outer sleeve move distally relative to the inner shaft. In other words, one of the inner shaft and outer sleeve moves relative to the other of the inner shaft and the outer sleeve upon operation of the adjustment mechanism.

The outer sleeve 150 is configured as best shown in FIGS. 1, 2, 3A, 3B and 8. The outer sleeve 150 is an annular sleeve and/or substantially cylindrical and open at its proximal and distal ends. The proximal end of the outer sleeve 150 is sized to be received by the opening in the distal portion of the housing 120. The outer sleeve 150 includes a circumferential shoulder 154 about its proximal end configured to abut the distal end of the housing. The midportion of the outer sleeve includes a second circumferential shoulder 156 that includes a through hole 152 configured to receive a shaft therein, as further described below. A third circumferential shoulder 158 is positioned about the distal end of the outer sleeve 150 for abutting the proximal side of the base 180 of the clamp 170. Both the proximal end and distal end of the outer sleeve include at least one flat surface 157, 159 for engaging corresponding unillustrated flat surface(s) in the distal opening of the housing and the proximal opening of the clamp base 180, respectively. Constructed and arranged as such, the flats on the outer sleeve prevent the outer sleeve from rotating relative to the housing and the clamp base.

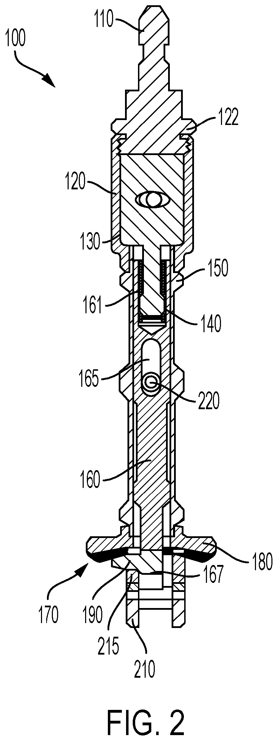

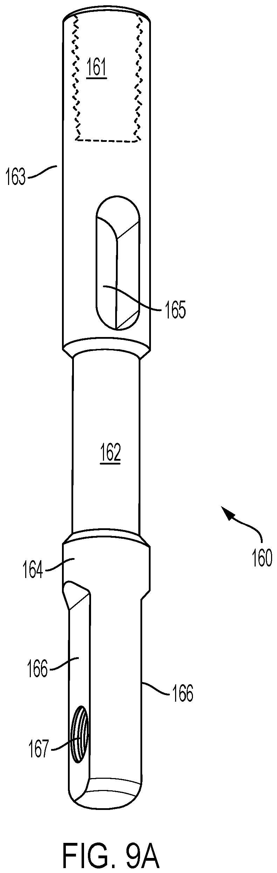

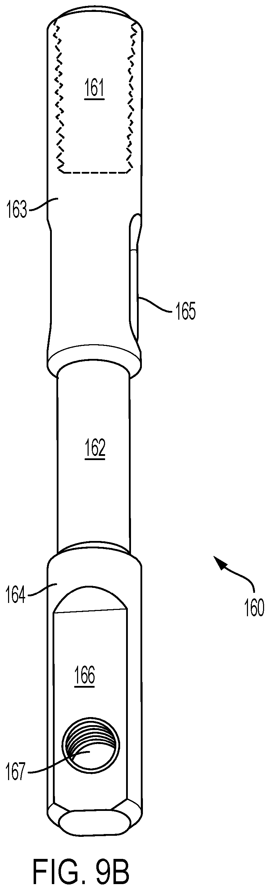

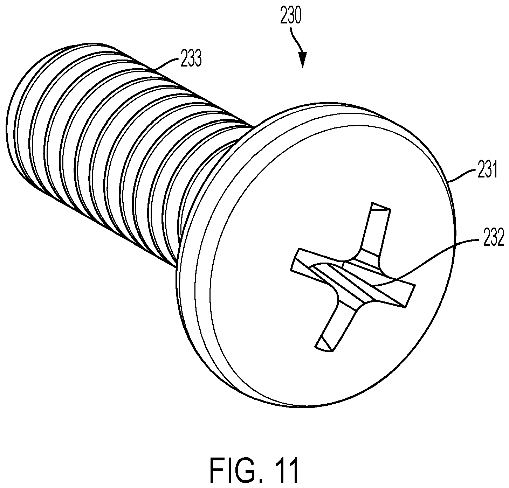

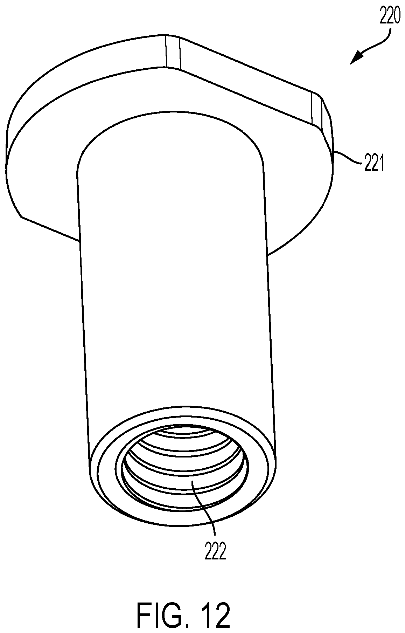

The inner shaft 160 is configured as best shown in FIGS. 2, 3, 9A and 9B. The inner shaft 160 is substantially cylindrical. As illustrated in FIGS. 2, 9A and 9B, the proximal end of the inner shaft 160 is threaded, e.g., it includes an internally threaded counterbore 161 for engaging with the threaded stem 140 of the adjustment mechanism 130. In addition, the inner shaft 160 includes a through slot 165 configured to receive a shaft (FIGS. 11 and 12) which is inserted through the hole 152 in the outer sleeve 150, thereby slidably connecting the inner shaft to the outer sleeve for a predetermined distance defined by the length of the through slot 165. In other words, the shaft moves within the slot 165. A mid-portion 162 of the inner shaft 160 can be configured to have a smaller diameter than proximal and distal portions 163, 164 thereof. The distal portion 164 of the inner shaft 160 includes two opposing flat surfaces 166 oriented at about 90 degrees from an open face of the through slot 165 in the proximal portion 163 of the inner shaft 160. The distal portion 164 of the inner shaft includes a threaded through hole 167, the function of which is described below.

The clamp 170 comprises the base 180 and the foot 190, which are configured as best shown in FIGS. 1, 2, 3A, 3B, 10A, 10B, 10C, 13, 14A and 14B. The base 180 has a concave distally directed face, which is substantially V-shaped in two directions e.g., in the anterior-posterior direction and the medial-lateral direction. The distally directed face of the base 180 can be textured, for example, to include ribs, teeth, knurling, protrusions, grooves, ridges, dimples, spikes, or a combination thereof. The base 180 also includes a through hole 181 for allowing the passage of the distal portion 164 the inner shaft 160 therethrough.

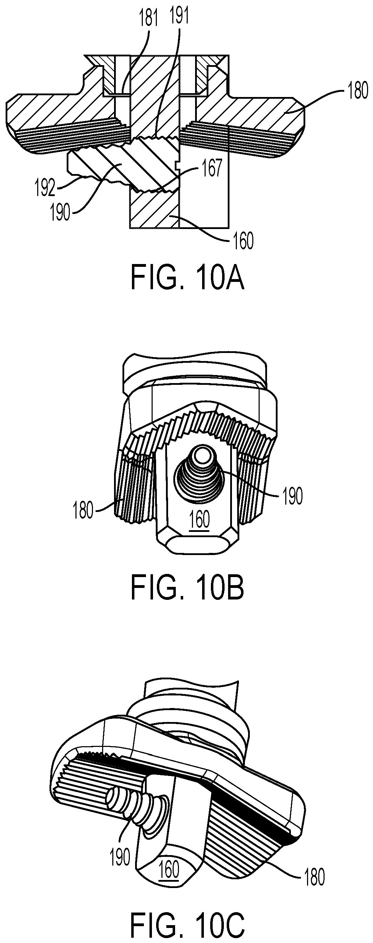

The foot 190 is threaded 191 for about one half of its length at its base and tapered 192 about its other half. The tapered portion can be textured, for example, to include ribs, teeth, knurling, protrusions, grooves, ridges, dimples, spikes, or a combination thereof. As shown in FIGS. 2 and 10A, the threaded through hole 167 in the inner shaft 160 is configured to receive the correspondingly threaded base 191 of the foot 190 of the clamp such that the tapered portion 192 of the foot extends laterally outwardly from the inner shaft. The foot is sized and configured to extend a length about 1/3, ½, ¾ a longitudinal length of the base. The base 180 of the clamp 170 is attached to the outer sleeve 150 prior to the insertion of the foot 190 through the inner shaft. FIG. 10A shows the foot 190 threaded into the threaded hole 167 of the inner shaft 160 and having its tapered and textured portion 192 protruding laterally. FIG. 10B shows the textured and tapered portion of the foot protruding from the inner shaft 160 and the base 180 having a concave face that is substantially V-shaped in two directions. FIG. 10C shows the clamp 170 wherein the proximal aspect of the base 180 abuts the distal end of the outer sleeve 150, the base circumferentially surrounding the inner shaft 160 and having a concave face substantially V-shaped in two directions.

The shaft is constructed as shown in FIG. 11. In the present exemplary example, the shaft comprises a screw 230 and a pin 220. The screw includes a head portion 231 with a tool-receiving socket 232, and a threaded shaft portion 233. The pin 220 is constructed as shown in FIG. 12. The pin includes an enlarged should portion 221 and a internally threaded bore 222.

To assemble the implant extractor 100, the through slot 165 of the inner shaft 160 is first aligned with the through hole 152 of the outer sleeve 150. The threaded pin 220 and the pan head screw 230 are then inserted in opposite sides of the through hole 152 and the through slot 165 and the pan head screw is threaded into the threaded pin to form the shaft or in other words a unitary pin, thereby connecting the outer sleeve 150 and the inner shaft 160 for relative axial motion. The distal end of the housing 120 is then placed onto the proximal end of the outer sleeve 150 and the adjustment mechanism 130 is inserted into the housing until the threaded stem 140 of the adjustment mechanism comes into engagement with the internally threaded bore 161 of the inner shaft 160. At this point the proximal portion 131 of the adjustment mechanism 130 is rotated to thread the threaded stem 140 into the threaded bore 161 an amount sufficient to connect the adjustment mechanism with the inner shaft. Thereafter, the quick connect 100 is threaded into the proximal end of the housing, the clamp base 180 is placed onto the distal end of the outer sleeve and the clamp foot 190 is threaded into the inner shaft.

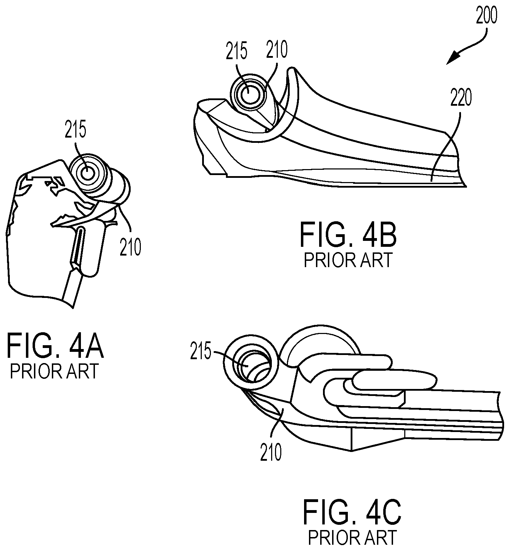

The process of using the implant extractor 100 to remove an elbow implant (e.g., a humeral and/or an ulnar implant) from bone, is as follows. It is understood that the implant extractor 100 may be used to extract other types of implants from other bones. As shown in FIGS. 2 and 4A-4C, the articular end of the implant 210 is exposed such that a circular opening 215 in the implant is accessible. The foot 190 of the clamp 170 is inserted into the circular opening 215. Owing to its taper, the foot 190 of the implant extractor 100 can accommodate various diameter implant openings. The adjustment mechanism 130 is then tightened by rotation of the proximal portion 131 resulting in e.g., motion of the outer sleeve 150 towards the implant while the inner shaft 160 and foot 190 remain fixed on the implant 210. In other words, as the adjustment mechanism is rotated, it causes one of the outer sleeve and inner sleeve to move relative to the other of the outer sleeve and inner sleeve. The result is stable and firm clamping of the implant 210 between the base 180 and foot 190 of the clamp 170. The joined pan head screw 230 and threaded pin 220 are slidable within the through slot 165 while being captured by the through hole 152 which prevents torque on the implant as the adjustment mechanism 130 is rotated.

With the implant clamped between the base 180 and the foot 190 of the clamp 170, the user pulls on the implant extractor 100 upwardly or away from the joint. If manually pulling upwardly on the implant extractor is insufficient to extract the implant from surrounding bone, a strike plate or slide hammer can be attached to the quick connect 110 of the implant extractor to facilitate removal of the implant.

It will be appreciated by those skilled in the art that changes could be made to the exemplary embodiments described above without departing from the broad inventive concept thereof. It is to be understood, therefore, that this disclosure is not limited to the particular embodiments disclosed, but it is intended to cover modifications within the spirit and scope of the subject disclosure as defined by the appended claims.

## Claims

1. An implant extractor (100) comprising a housing (120), **characterized by**:
an adjustment mechanism (13) received within the housing;
an outer sleeve (150) extending from the housing;
an inner shaft (160) received in the outer sleeve and having a proximal end operatively engaged with the adjustment mechanism; and
a clamp (170) operatively engaged with distal ends of the inner shaft and the outer sleeve.

2. The implant extractor of claim 1, wherein the inner shaft includes a threaded proximal end (161).

3. The implant extractor of claim 1 or claim 2, wherein the adjustment mechanism comprises a threaded stem (140) for engaging the threaded proximal end of the inner shaft.

4. The implant extractor of any one of claims 1-3, wherein the inner shaft includes a slot (165) and the outer sleeve includes a shaft (220, 230) for moving within the slot.

5. The implant extractor of any one of claims 1-4, wherein the outer sleeve includes a shoulder (154) about its proximal end abutting the housing.

6. The implant extractor of any one of claims 1-5, wherein the outer sleeve includes a shoulder (158) about its distal end abutting the clamp.

7. The implant extractor of any one of claims 1-6, wherein the clamp comprises a base (180) and a foot (190).

8. The implant extractor of any one of claims 1-7, wherein the base has a concave face.

9. The implant extractor of any one of claims 1-8, wherein the concave face is a substantially V-shaped face, or wherein the concave face is substantially V-shaped in two directions.

10. The implant extractor of any one of claims 1-9, wherein the concave face includes a textured surface including ribs, teeth, knurling, protrusions, grooves, ridges, dimples, spikes, or a combination thereof.

11. The implant extractor of claim 7, wherein the foot is tapered (192).

12. The implant extractor of claim 7, wherein the foot includes a textured surface (192).

13. The implant extractor of any one of claims 1-12, wherein the housing has a window (121) in communication with a proximal portion of the adjustment mechanism.

14. The implant extractor of any one of claims 1-13, further comprising a quick connect (110) attached to a proximal end of the housing.

15. The implant extractor of any one of claims 1-14, wherein one of the outer sleeve and the inner shaft moves relative to the other of the outer sleeve and the inner shaft upon operation of the adjustment mechanism, or wherein the outer sleeve moves relative to the inner shaft upon operation of the adjustment mechanism.
